(19) Europäisches Patentamt / European Patent Office / Office européen des brevets

(11) **EP 3 650 851 B1**

(12) **EUROPEAN PATENT SPECIFICATION**

(45) Date of publication and mention
of the grant of the patent:
**07.09.2022  Bulletin 2022/36**

(21) Application number: **18306451.8**

(22) Date of filing: **06.11.2018**

(51) International Patent Classification (IPC):
*G01N 33/00* (2006.01)        *A61B 5/00* (2006.01)
*A61B 5/0205* (2006.01)        *A61B 5/11* (2006.01)
*G04G 21/02* (2010.01)

(52) Cooperative Patent Classification (CPC):
**G01N 33/0006; A61B 5/0205; A61B 5/1118;
A61B 5/681; G01N 33/0037; G04G 21/02;
G04G 21/025;** A61B 2560/0223; A61B 2560/0242;
Y02A 50/20

(54) **CALIBRATION OF AN AIR QUALITY SENSOR IN A DEVICE**

KALIBRIERUNG EINES LUFTQUALITÄTSSENSORS IN EINER VORRICHTUNG

ÉTALONNAGE D'UN CAPTEUR DE QUALITÉ DE L'AIR DANS UN DISPOSITIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**13.05.2020  Bulletin 2020/20**

(73) Proprietor: **Withings
92130 Issy les Moulineaux (FR)**

(72) Inventors:
• **LEGER, Charlotte
  92120 MONTROUGE (FR)**
• **BESSIS, Charlotte
  75015 PARIS (FR)**

• **OUESLATI, Ianis
  92170 VANVES (FR)**
• **BESNARD, Marc
  75017 PARIS (FR)**

(74) Representative: **Plasseraud IP
66, rue de la Chaussée d'Antin
75440 Paris Cedex 09 (FR)**

(56) References cited:
**WO-A1-2017/207594      CN-U- 204 086 834
US-A- 4 590 789          US-A1- 2011 197 649
US-A1- 2017 351 221      US-B1- 6 774 613**

## Description

### FIELD OF THE DISCLOSURE

The present disclosure concerns a wearable device, for example a wrist watch or an activity tracker, with an air quality sensor.

### BACKGROUND OF THE DISCLOSURE

It is known to embed an air quality sensor into a device like a wrist watch, for example from US 2017/351221 A1 or CN 204086834 U.

The air quality sensor can detect and monitor the presence of air pollution in a surrounding area, for both indoor and outdoor environments. It can provide useful information to a user on the air quality that may negatively impact his health, and cause harmful effects such as cold symptoms or respiratory diseases.

In such a device, the air quality sensor can produce an electric current according to a concentration level of some compounds present in the air.

However, the electrical current may also vary according to other parameters, such as humidity, temperature and aging of other components constitutive of the device which may release gas affecting the accuracy of the air quality sensor located nearby.

It is therefore necessary to recalibrate the sensor on a regular basis. In the absence of recalibration, the air quality sensor is likely to output inaccurate measures that may mislead the user. Calibration of gas sensors is generally known, for example from WO 2017/207594 A1, US 6774613 B1, US 2011/197649 A1, or US 4590789 A.

However, calibration process is usually difficult and burdensome to implement for a user.

Therefore, there remains a need to provide a device with an air quality sensor able to provide reliable air quality measurements, whatever its conditions of use, and that remains easy to use in the context of everyday life.

### SUMMARY OF THE DISCLOSURE

The present disclosure relates to a device comprising a cavity defining an internal volume, an air quality sensor being adapted to measure a concentration of at least one air compound present in the internal volume. The cavity can be alternatively in:

- an open state in which air can enter into the cavity from the outside of the cavity, and
- a closed state in which no air can circulate between the outside and the inside of the cavity,

the air quality sensor being adapted to be calibrated when the cavity is in the closed state once the internal volume no longer comprises the air compound or comprises a concentration of the air compound that is below a predefined threshold, wherein the device further comprises an accelerometer adapted to detect a movement of the user, the air quality sensor being adapted to be calibrated when the accelerometer does not detect any movement of the user, wherein the device is a wearable device, such as a watch.

Thanks to these dispositions, it is possible to perform a reliable measurement of the air compound.

In various embodiments according to the present disclosure, use may also possibly be made of one and/or the other of the following dispositions, taken separately or in combination, according to which:

- the cavity comprises an opening so that air can exclusively enter the cavity through the opening, the cavity being elsewhere isolated from the device ;
- the air quality sensor is adapted to produce an electric current by consuming the air compound present in the internal volume of the cavity,
- the air compound is $NO_2$,
- the internal volume of the cavity is less than 0.3 cm$^3$, less than 0.2 cm$^3$, less than 0.1 cm$^3$, or even less than 0.05 cm$^3$;
- the cavity comprises a first grid and a second grid, the second grid being adapted to move relative to the first grid so that the cavity can be in the open state or in the closed state;
- each of the first and second grids comprises a plurality of through apertures, wherein at least some of the through apertures of the first and second grids face each other when the cavity is in the open state and wherein the through apertures of the first and second grids are offset to one another when the cavity is in the closed state;
- the cavity comprises a door, the door being adapted to move relative to the opening so that the cavity can be in the open state or in the closed state;
- the cavity comprises a bottom portion and a top portion, wherein the bottom portion comprises a first hole and a second hole, the air quality sensor being located in the first hole, the cavity and the air quality sensor being sealed together in an airtight manner by using at least one gasket, such that the air quality sensor is in direct contact with the air present in the internal volume of the cavity. The present disclosure also relates to a method for calibrating an air quality sensor of the device according to any of appended claims 1 to 9, comprising at least the steps of:
- switching the cavity from the open state to the closed state;
- waiting for the internal volume to no longer comprise the air compound or to comprise a concentration of the air compound that is below a predefined threshold; and
- calibrating the air quality sensor.

## BRIEF DESCRIPTION OF THE DRAWINGS

Other features of the disclosure appear from the following detailed description, given by way of non-limiting example, and with reference to the accompanying drawings, in which:

- Figure 1 shows an exploded view of the device according to the present disclosure,
- Figure 2 shows a perspective view of an internal part of the device of figure 1 according to a first embodiment,
- Figure 3 shows an exploded view of the internal part of figure 2,
- Figure 4 shows a perspective view of a cavity of the internal part of figures 2 and 3, the cavity comprising a first grid,
- Figure 5A shows front view of a second grid according to the first embodiment,
- Figure 5B shows a cross section of the second grid of figure 5A according to cross-sectional plane VB-VB,
- Figure 6 shows a view of the cavity of figure 3 in which the cavity is in a closed state,
- Figure 7 shows a view of the cavity of figure 3 in which the cavity is in an open state,
- Figure 8 shows an exploded view of the internal part of a device of figure 1 according to a second embodiment,
- Figure 9 shows a perspective view of a cavity of the internal part of figure 8,
- Figure 10 shows front view of a door according to the second embodiment,
- Figure 11 shows a view of the cavity of figure 8 in which the cavity is in a closed state,
- Figure 12 shows a view of the cavity of figure 8 in which the cavity is in an open state, and
- Figure 13 shows a perspective top view of the internal part of figure 12.

In the figures, the same references denote identical or similar elements.

## DETAILLED DESCRIPTION OF THE DISCLOSURE

DEVICE

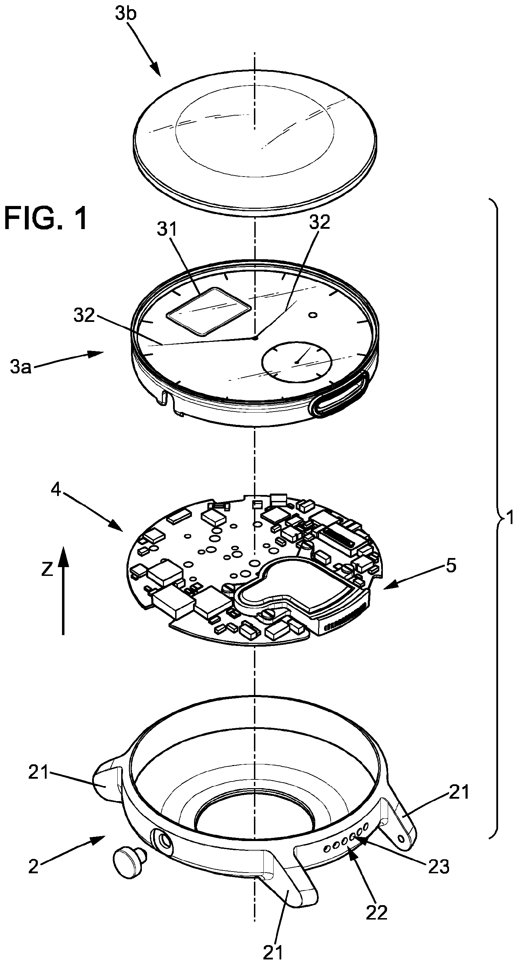

Figure 1 shows a device 1 according to the present disclosure. The device 1 is a wearable device, such as a watch, a wrist watch or an activity tracker.

However, the device 1 can also be any other wearable device, such as glasses, a necklace, a ring, etc.

As illustrated on figure 1, the device 1 may comprise a casing 2, a dial plate 3a and a top cover 3b of the dial plate 3a. The casing 2, the dial plate 3a and the top cover 3b are arranged one on top of another in a vertical direction Z. The thickness of the device 1 is thus defined along this vertical direction Z.

The device 1 can be a digital watch, such that the dial plate 3a comprises a display 31. As a variant or complementarily, the device 1 may be an analog type watch, such that the dial plate 3a comprises stick-like hands 32 with a traditional clock face for instance.

To be attached to a wrist of a user, the casing 2 may comprise projections 21 for attachment to a wristband (not illustrated) as known *per se* and thus not described in more details.

As illustrated on figure 1, the device 1 can have a round shape. However, other shapes, such as square or rectangle, are also possible. Similarly, the device 1 can be relatively small, as for a watch for example, or can have bigger dimensions when it comes to objects having larger dimensions, as for a weight scale for another example.

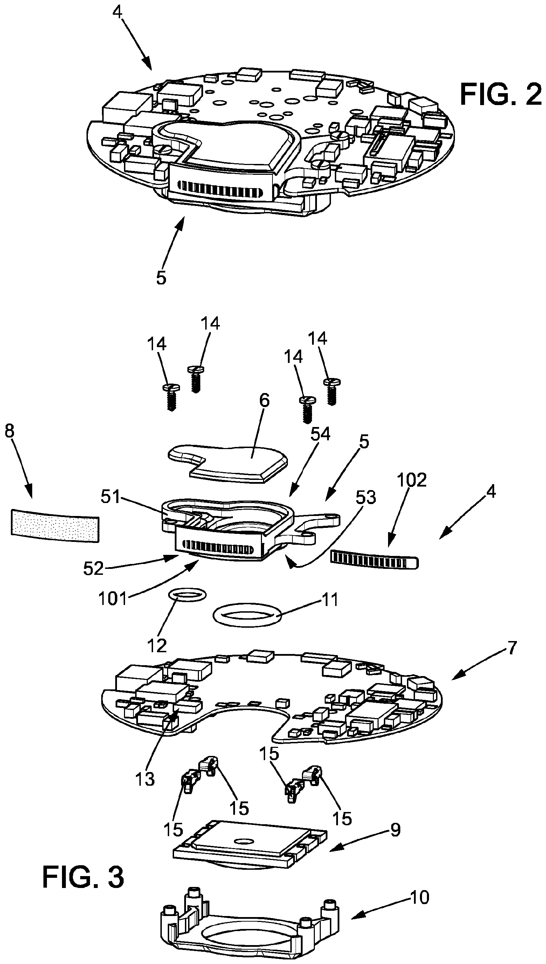

The device 1 also comprises an internal part 4 illustrated on figure 2. As illustrated on figure 1, the internal part 4 can be sandwiched between the casing 2 and the dial plate 3a.

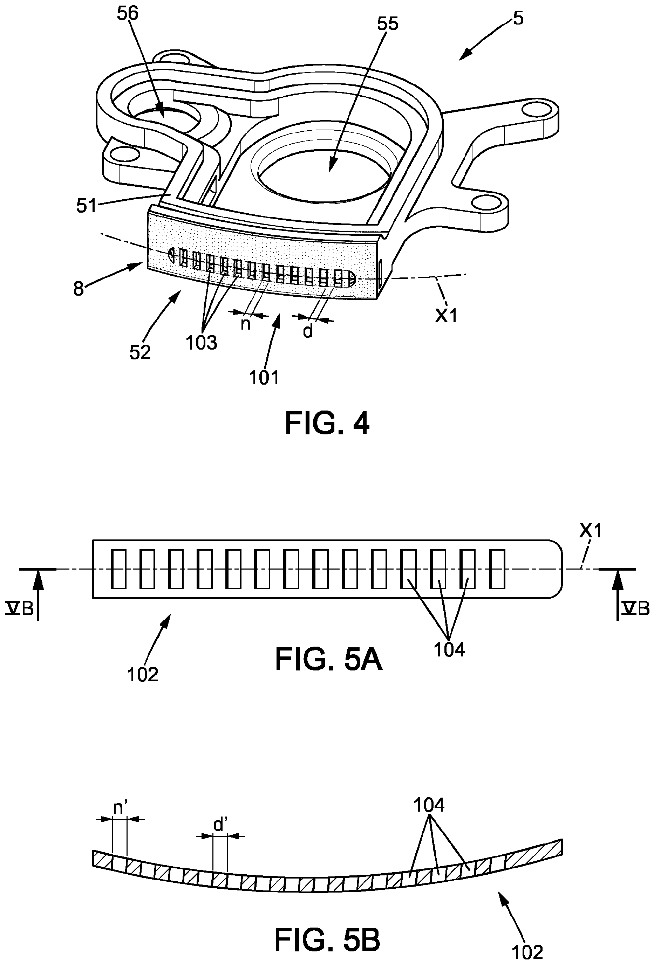

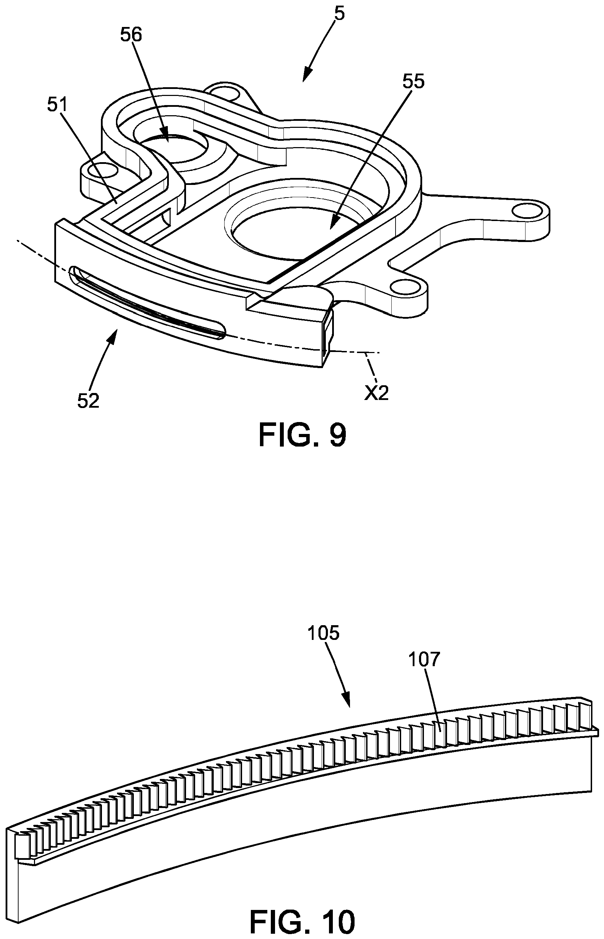

As illustrated more particularly on figures 4 and 9, the internal part 4 comprises a cavity 5. The cavity 5 may be molded, in particular as a single piece.

The cavity 5 comprises a wall 51 and an opening 52.

The wall 51 forms at least the lateral portion of the cavity 5.

The cavity 5 also comprises a bottom portion 53 and a top portion 54.

The bottom portion 53 can comprise a first hole 55 and a second hole 56. As illustrated on figures 4 and 9, the holes 55, 56 can have a circular shape.

The top portion 54 can be closed by a cover 6.

The cavity 5 defines and delimits an internal volume. The internal volume is suited and intended to receive an air volume coming from the outside of the cavity 5, and more precisely from the outside of the device 1, through the opening 52. The cavity 5 may take any form, notably cylindrical, parallelepipedal or other so as to form the internal volume.

The terms "internal", "inside" and "external", "outside" when applied to the cavity 5 reflect the fact that the cavity 5 delimits the internal volume comprising a quantifiable amount of air, distinguishable from the air present in the surrounding of the device 1 or present elsewhere in the device 1. It is therefore with reference to this situation that these terms should be understood.

The cavity 5 can be arranged on, or fixed to, a printed circuit board 7. The printed circuit board 7 can comprise a control unit, an oscillator, a battery either conventional or rechargeable (not illustrated), or any known elements for the functioning of the device 1.

In order for the internal volume of the cavity 5 to be in fluid communication with the outside of the device 1, the casing 2 can comprise a perforated area 22, wherein through bores 23 opens on the cavity 5 and the outside of the device 1.

The through bores 23 may be aligned in the perforated area 22, facing the opening 52 of the cavity 5.

As illustrated on figure 1, the casing 2 comprises six through bores 23. Each through bore 23 may have a diameter comprised between 0.5 mm (millimeter) and 1.5 mm, for example about 1 mm.

In an embodiment, the device 1 can also comprise a membrane 8 which is impermeable to water but permeable to air. The water-impermeable membrane 8 is placed facing the opening 52 so that air entering the cavity 5 passes through the membrane 8.

As illustrated more particularly on figures 3, 4, 6 and 7, the water-impermeable membrane 8 can be placed in front of the opening 52 toward the outside of the cavity 5.

As a variant, the water-impermeable membrane 8 may as well be inside the cavity 5 on the inside face of the opening 52.

As another variant, the water-impermeable membrane 8 may also be placed on the internal or external side of the perforated area 22. This way, the water-impermeable membrane 8 can ensure that the whole device 1 is waterproof.

The water-impermeable membrane 8 prevents the ingress of water inside the cavity 5. This way, the user can for example immerse briefly the device 1 in water without any damage.

The water-impermeable membrane 8 may be formed as a thin wall manufactured in synthetic material having a microporous structure.

AIR QUALITY SENSOR

The device 1 comprises an air quality sensor 9. The air quality sensor 9 is arranged in the cavity 5 of the internal part 4.

"The air quality sensor is arranged in the cavity" means that the air quality sensor 9 is located inside, opens on, or is at least is in fluid communication with the internal volume of the cavity 5.

More particularly, as illustrated on figure 3, the air quality sensor 9 is placed in the first hole 55 of the cavity 5. This way, the air quality sensor 9 is in direct contact with the air present in the internal volume of the cavity 5.

The air quality sensor 9 is sealed with the cavity 5 in an airtight manner with a gasket 11. To ensure that the air quality sensor 9 is sealed with the cavity 5, the device 1 can comprise a supporting element 10. The supporting element 10 holds the air quality sensor 9 against the cavity 5.

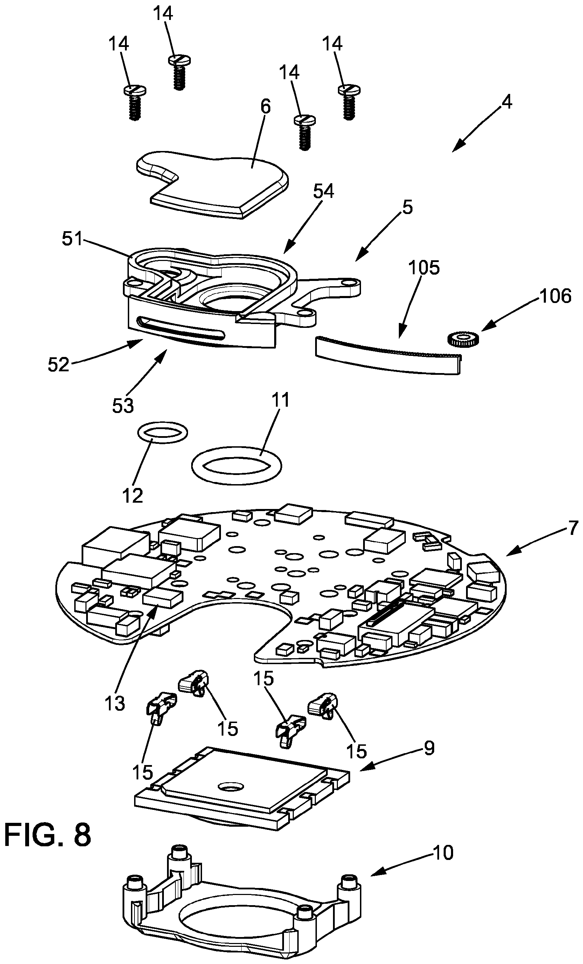

More precisely, the cavity 5 can be fixedly attached to the supporting element 10. As illustrated on figures 3 and 8, the device 1 may comprise screws 14 that go through the cavity 5 and the printed circuit board 7 and that are fixed to the supporting element 10.

Spring elements 15 are compressed between the printed circuit board 7 and the air quality sensor 9. The spring elements 15 can be electrical conductors that ensure electrical contact between the air quality sensor 9 and the printed circuit board 7.

As a variant, to ensure that the air quality sensor 9 is sealed with the cavity 5, the air quality sensor 9 may be soldered to the printed circuit board 7 and the cavity 5 may be screwed on the printed circuit board 7, thus compressing the gasket 11.

The device 1 can also comprise other types of sensors, such as a temperature sensor, a humidity sensor, a pressure sensor, or the like.

As illustrated on figures 3 and 8, the device 1 comprises a humidity sensor 13 arranged in the cavity 5 of the internal part 4. The humidity sensor 13 is placed in the second hole 56 of the cavity 5. The humidity sensor 13 is sealed with the cavity 5 in an airtight manner with a gasket 12. The gasket 12 can seal the humidity sensor 13 with the cavity 5 in the same manner as the gasket 11 seals the air quality sensor 9 with the cavity 5.

Air can exclusively circulate between the outside and the inside of the cavity 5 through the opening 52, the cavity 5 being elsewhere isolated from the device 1. In particular, the cavity 5 is isolated from the other elements of the internal part 4 of the device 1, and more precisely from the other elements of the printed circuit board 7.

The air quality sensor 9 is adapted to measure a concentration of one or several air compounds in the vicinity of the device 1.

"In the vicinity of the device" means that the air compound(s) measured by the device 1 are not substantially different from the air compound(s) that the user of the device 1 is breathing or that surrounds the device 1.

The one or several air compounds are of interest. This way, the device 1 is adapted to detect compounds in the general environment of the device 1 and/or detect some chemical species present in the breath of the user when the user exhales or blows toward the device 1. The air quality sensor 9 is particularly suited to measure a concentration of some air compounds which may have an impact on human health (also called "pollutants").

The air quality sensor 9 may be adapted to measure a concentration of an air compound chosen among $NO_2$ (nitrogen dioxide), $O_3$ (ozone), $NO$ (nitric oxide), $CO$ (carbon monoxide), $SO_2$ (sulphur dioxide), $CO_2$ (carbon dioxide), $H_2S$ (hydrogen sulfide), $Cl$ (chlorine), benzene, $CH4$ (methane), $NH_4$ (ammonia), $(CH_3)_2CO$ (acetone) and alcohol vapours. The air compound can be a volatile organic compound.

When the air quality sensor 9 is used to detect some chemical species present in the breath of the user for instance, the air compound can be more precisely $NH_4$ (ammonia), $(CH_3)_2CO$ (acetone) and alcohol vapours.

The air quality sensor 9 may be adapted to measure a concentration of only one air compound or of several air compounds, in particular simultaneously. For instance, the air quality sensor 9 may be adapted to measure a concentration of two air compounds, such as those mentioned-above. The air quality sensor 9 may be reactive both to $NO_2$ and $O_3$.

According to an embodiment, the air quality sensor 9 can be an amperometric sensor. The air quality sensor 9 produces an electric current when a potential is applied between electrodes.

A first chemical occurs at least at one working electrode (or catalyst) when in contact with the air, which takes place with regard to at least one auxiliary electrode (or reference).

For example, if the air quality sensor 9 is adapted to measure a concentration of CO, the first chemical reaction may be:

$CO + H_2O <=> CO_2 + 2e- + 2H+$ (electrons are released in the working electrode)

According to another example, if the air quality sensor 9 is adapted to measure a concentration of $NO_2$, the first chemical reaction may be:

$NO_2 + 2e- + 2H+ <=> NO + H_2O$ (electrons are captured in the working electrode)

These chemical reactions are purely illustrative and non-limitative. More complex chemical reactions involving a larger number of chemical species are also possible.

Measurement of concentration of the air compound can also depend on other parameters, such as the ambient temperature, ambient humidity (more particularly measured by the humidity sensor 13 described above), ambient pressure or the electric intensity flowing through the air quality sensor 9.

Electrolyte may be provided between two or more electrodes to transport the ions produced by the chemical reactions from one electrode to the other electrode.

The air quality sensor 9 may have a low electric consumption, for example an average electric consumption when powered of less than 10 $\mu$A (microampere), or less than 2 $\mu$A. The electric current produced by the air quality sensor 9 can be comprised between 0.1 nA (nanoAmp) and 20 nA depending on the concentration of the air compound measured.

The lifetime of the battery of the device 1 can be sufficient to ensure a sufficiently long working life of the air quality sensor 9.

By way of example, the battery of the device 1 may have a capacity of 120 mAh (milliampere hour), with an autonomy of 25 days or 600 hours without using the air quality sensor 9. Its average electric consumption is 200 $\mu$A.

By considering that the average consumption of the air quality sensor 9 is 10 $\mu$A, the average electric consumption of the air quality sensor 9 is equal to 5% of the average electric consumption of the device 1 without using the air quality sensor 9. The device 1 may thus have an autonomy of around 24 days or around 570 hours when powering the air quality sensor 9.

The air quality sensor 9 is connected to the control unit of the device 1 which collects and processes the measurements of concentrations of the air compounds.

Such measurements can then be provided to the user, for instance on the display 31 of the device 1. As an alternative or complementarily, the device 1 may wirelessly communicate with another external terminal (not illustrated), such as a smartphone or a server, to send the collected measurement data.

OPENING AND CLOSING OF THE CAVITY

Measurements made by the air quality sensor 9 can become out of calibration over time for a number or reasons.

For instance, external reasons, such as humidity and temperature, or internal reasons such as aging of components (e.g. electronic component, battery or coating material), may affect the accuracy of the air quality sensor 9.

It is therefore necessary to recalibrate the air quality 9 on a regular and/or periodic basis.

To this end, the cavity 5 can be alternatively in an open state or a closed state.

In the open state, air can enter into the cavity 5, so that the air present in the internal volume is constantly renewed from the outside of the cavity 5, and more particularly from the outside of the device 1.

In the closed state, no air can circulate between the outside and the inside of the cavity 5. The air present in the internal volume cannot be renewed and remains trapped in the cavity 5 as long as the cavity does not switch to the open state. The cavity 5 is hermetically sealed or airtight with respect to the air located outside the cavity 5, whether this air is located elsewhere inside the device 1 or outside the device 1.

In the closed state, concentration of the air compound can decrease over time, in particular since air cannot enter anymore into the cavity 5.

More precisely, the air quality sensor 9 is adapted to consume the air compound that is present in the internal volume of the cavity 5.

When the cavity 5 is in the closed state, the air quality sensor 9 can thus consume the entire air compound present in the internal volume of the cavity 5, so that the cavity 5 no longer comprises the air compound or comprises the air compound having a concentration that is below a predefined threshold, such as the detection threshold of the air quality device 9.

When the air compound is $NO_2$, such predefined threshold can be 5 ppb (parts per billion), 1 ppb or even 0.1 ppb.

For instance, the air quality sensor 9 can be adapted to measure the concentration of $NO_2$ according to the chemical reaction mentioned above, so that the air compound $NO_2$ can be progressively consumed by chemical reaction by the air quality sensor 9 when the cavity 5 is in the closed state, so that no $NO_2$ is finally left in the internal volume of the cavity 5 or only in trace amounts.

The air quality sensor 9 can consume the air compound in less than 8 hours, or in less than 4 hours, or in less than one hour, or even in less than 30 minutes. Consumption time of the air compound may depend on the air quality sensor parameters and on the size of the internal volume of the cavity 5.

To this end, the internal volume of the cavity 5 can be less than 0.3 $cm^3$ (cubic centimetre), less than 0.2 $cm^3$, less than 0.1 $cm^3$, or even less than 0.05 $cm^3$.

Then, the air quality sensor 9 can be calibrated. Calibration can encompass several possibilities, such as setting a new baseline measurement or the sensitivity of the air quality sensor 9. Calibration can also permit to obtain a calibration function in the form of a curve, a look-up table, or any other form in order to describe the linear or non-linear relation between the air compound concentration and the measurements to be performed by the air quality sensor 9.

Advantageously, the air quality sensor 9 can set a new baseline measurement (or a new "zero"). Every new measurement of the air quality sensor 9 can then be estimated by being compared with this new baseline measurement. This ensures a good reliability and accuracy of the air quality sensor 9 over time.

In particular, the air quality sensor 9 may be adapted to measure several air compounds but can be calibrated according to only one air compound.

When calibration is implemented, other parameters, such as the ambient temperature, ambient humidity, ambient pressure or the electric intensity flowing through the air quality sensor 9, may also be measured or set as reference values.

The material of the cavity 5 may also be advantageously chosen not to affect the measurements made by the air quality sensor 9. For instance, when the air quality sensor 9 is adapted to measure a concentration of $NO_2$, the cavity 5 may be made of polytetrafluoroethylene (PTFE).

We here below describe two embodiments of the cavity 5 so that it can be in the open or closed state.

FIRST EMBODIMENT OF THE DEVICE

According to a first embodiment, the device 1 comprises a first grid 101 and a second grid 102. The first grid 101 is fixed relative to the cavity 5. In particular, the first grid 101 may be integral with the cavity 5. The first grid 101 is located at the inlet of, or forms, the opening 52 of the cavity 5.

The second grid 102 is adapted to move relative to the first grid 101 so that the cavity 5 can be in the open state or in the closed state. More precisely, the second grid 102 is adapted to slide relative to the first grid 101 along a longitudinal direction X1. The longitudinal direction X1 can be perpendicular to the vertical direction Z.

To move the second grid 102, the device 1 may comprise an actuator or motor (not illustrated) adapted to allow small displacements. The motor can for instance be piezoelectric, electromagnetic or it can be a stepper motor.

Each of the first and second grids 101, 102 comprises a plurality of through apertures 103, 104. The through apertures 103, 104 of the first and second grids 101, 102 are respectively spaced apart regularly in the longitudinal direction X1.

As illustrated on figures 4, 5A and 5B, each of the first and second grids 101, 102 comprises fourteen through apertures 103, 104. However, this is not limitative and the first and second grids 101, 102 can comprise less or more through apertures 103, 104.

The through apertures 103, 104 of the first and second grids 101, 102 can be of rectangular shapes. However, other shapes are possible.

As illustrated on figure 4, the through apertures 103 of the first grid 101 have a width n in the longitudinal direction X1. The width n of the through apertures 103 of the first grid 101 can be less than 1 mm (millimeter), or even less than 0.5 mm. The width n can be for instance equal to 0.3 mm.

The through apertures 103 of the first grid 101 are spaced apart regularly from each other by a distance d (also called "pitch").

Similarly, as illustrated on figures 5A and 5B, the through apertures 104 of the second grid 102 have a width n' in the longitudinal direction X1. The width n' of the through apertures 104 of the second grid 102 can be less than 1 mm (millimeter), or even less than 0.5 mm. The width n' can be for instance equal to 0.3 millimeters.

The through apertures 104 of the second grid 102 are spaced apart regularly from each other by a distance d'.

The widths n, n' and the distances d, d' are interrelated so that the cavity 5 can be opened or closed in a satisfactory manner by moving the second grid 102 relative to the first grid 101

Advantageously, the first and second grids 101, 102 are such that:

$$n' \geq n,$$

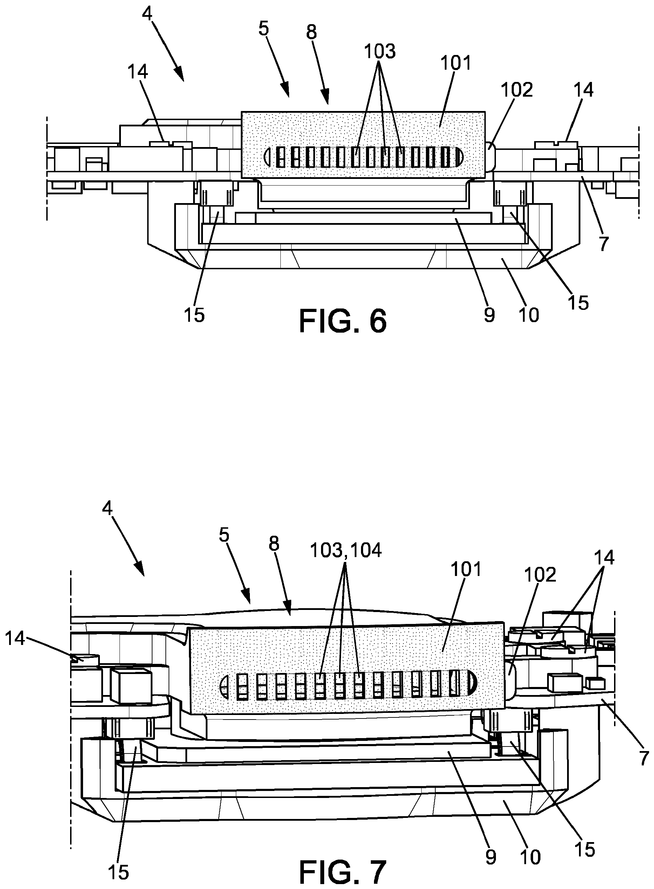

This way, in the open position, a through aperture 104 of the second grid 102 overlaps totally a though aperture 103 of the first grid 101. All or some of the though apertures 103, 104 face each other. The cavity 5 is thus open as illustrated on figure 7.

Advantageously, the first and second grids 101, 102 are such that:

$$d' \geq n \text{ and } d \geq n'$$

This way, in the closed position, the solid space between two consecutive through apertures 104 of the second grid 102 overlaps totally, or covers, a through aperture 103 of the first grid 101. Similarly, the solid space between two consecutive through apertures 103 of the first grid 101 overlaps totally, or covers, a through aperture 104 of the second grid 102. The through apertures 103, 104 of the first and second grids 101, 102 are offset to one another in the longitudinal direction X1. The cavity 5 is thus totally closed as illustrated on figure 6.

SECOND EMBODIMENT OF THE DEVICE

According to a second embodiment, the device 1 comprises a door 105. The door 105 is adapted to move relative to the opening 52 so that the cavity 5 can be in the open state or in the closed state. More precisely, the door 105 is adapted to slide relative to the opening 52 along a longitudinal direction X2. The opening 52 may consist in a single aperture which extends in the longitudinal direction X2.

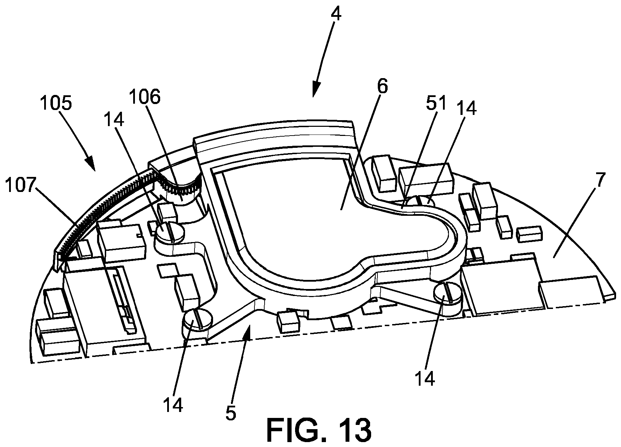

To move the door 105, the device 1 may comprise a toothed disk or a pinion 106. The door 105 comprises a rack 107 that can engage with the toothed disk 106, as illustrated more particularly on figure 13.

The door 105 is a full solid surface, which does not comprise any through apertures unlike the second grid 102 of the first embodiment described above.

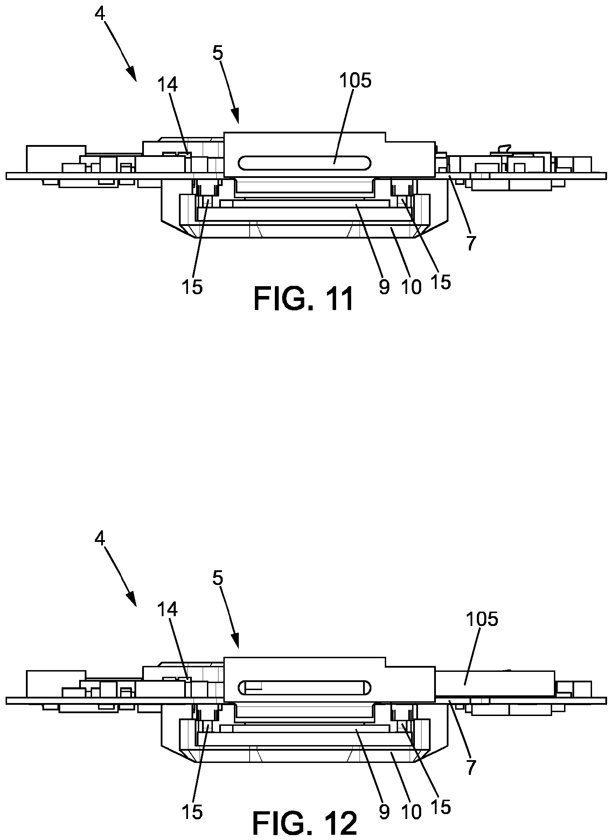

In the closed state, the door 105 faces the opening 52. The cavity 5 is thus totally closed as illustrated on figure 11.

In the open state, the door 105 does not cover the opening 52 by being slidably moved, preferably totally, on a side of the opening 52 along the longitudinal direction X2. The cavity 5 is thus open as illustrated on figure 12.

ADVANTAGES OF THE DISCLOSURE

Thanks to the fact that the cavity 5 can switch from the open state to the closed state, it is possible to recalibrate the air quality sensor 9 when the air compound to be measured has been totally, or almost totally, consumed in the internal volume.

This calibration is advantageously implemented when the user is unlikely to use the device 1 as well as the embedded air quality sensor 9 for a given period of time.

To this end, according to the present invention the device 1 comprises an accelerometer (not illustrated) adapted to detect a movement of the user. Calibration of the air quality sensor 9 is then implemented when the accelerometer does no detect any movement of the user.

Such calibration can for instance occur during a sleeping phase of the user or, in the embodiment wherein the device 1 is a wearable device, when the device 1 is not worn by the user.

Such calibration can also be repeated in a periodic manner. For instance, such calibration can occur at least once a month, or at least once a week, or even at least once a day. This way, it ensures that the air quality sensor 9 always output a reliable measurement of the concentration of the air compound.

**Claims**

1.  A device (1) comprising a cavity (5) defining an internal volume, an air quality sensor (9) being adapted to measure a concentration of at least one air compound present in the internal volume, wherein the cavity (5) can be alternatively in:

    - an open state in which air can enter into the cavity (5) from the outside of the cavity (5), and
    - a closed state in which no air can circulate between the outside and the inside of the cavity (5),

    the air quality sensor (9) being adapted to be calibrated when the cavity (5) is in the closed state and once the internal volume no longer comprises the air compound or comprises a concentration of the air compound that is below a predefined threshold, **characterized in that** the device further comprises an accelerometer adapted to detect a movement of the user, the air quality sensor (9) being adapted to be calibrated when the accelerometer does not detect any movement of the user, wherein the device (1) is a wearable device, such as a watch.

2.  The device according to claim 1, wherein the cavity (5) comprises an opening (52) so that air can exclusively enter the cavity (5) through the opening (52), the cavity (5) being elsewhere isolated from the device (1).

3.  The device (1) according to any of claims 1 to 2, wherein the air quality sensor (9) is adapted to produce an electric current by consuming the air compound present in the internal volume of the cavity (5).

4.  The device (1) according to any of claims 1 or 3, wherein the air compound is $NO_2$.

5.  The device (1) according to any of claims 1 to 4, wherein the internal volume of the cavity (5) is less than 0.3 cm$^3$, less than 0.2 cm$^3$, less than 0.1 cm$^3$, or even less than 0.05 cm$^3$.

6.  The device (1) according to any of claims 1 to 5, wherein the cavity (5) comprises a first grid (101) and a second grid (102), the second grid (102) being adapted to move relative to the first grid (101) so that the cavity (5) can be in the open state or in the closed state.

7.  The device (1) according to claim 6, wherein each of the first and second grids (101, 102) comprises a plurality of through apertures (103, 104), wherein at least some of the through apertures (103, 104) of the first and second grids (101, 102) face each other when the cavity (5) is in the open state and wherein the through apertures (103, 104) of the first and second grids (101, 102) are offset to one another when the cavity (5) is in the closed state.

8. The device (1) according to any of claims 1 to 5, wherein the cavity (5) comprises a door (105), the door (105) being adapted to move relative to the opening (52) so that the cavity (5) can be in the open state or in the closed state.

9. The device (1) according to any of claims 1 to 8, wherein the cavity (5) comprises a bottom portion (53) and a top portion (54), wherein the bottom portion (53) comprises a first hole ( 55) and a second hole (56), the air quality sensor (9) being located in the first hole ( 55), the cavity (5) and the air quality sensor (9) being sealed together in an airtight manner by using at least one gasket (11), such that the air quality sensor (9) is in direct contact with the air present in the internal volume of the cavity (5).

10. A method for calibrating an air quality sensor (9) of a device (1) according to any of claims 1 to 9, comprising at least the steps of:

- switching the cavity (5) from the open state to the closed state;
- waiting for the internal volume to no longer comprise the air compound or to comprise a concentration of the air compound that is below a predefined threshold; and
- calibrating the air quality sensor (9).

**Patentansprüche**

1. Vorrichtung (1), die einen Hohlraum (5), der ein Innenvolumen definiert, und einen Luftqualitätssensor (9), der geeignet ist, eine Konzentration wenigstens einer Luftverbindung, die in dem Innenvolumen vorhanden ist, zu messen, aufweist,

wobei der Hohlraum (5) alternativ sein kann:

- in einem offenen Zustand, in dem Luft von außerhalb des Hohlraums (5) in den Hohlraum (5) eintreten kann, und
- in einem geschlossenen Zustand, in dem keine Luft zwischen dem Äußeren und dem Inneren des Hohlraums (5) zirkulieren kann,

wobei der Luftqualitätssensor (9) geeignet ist, kalibriert zu werden, wenn der Hohlraum (5) in dem geschlossenen Zustand ist und sobald das Innenvolumen die Luftmischung nicht mehr aufweist oder eine Konzentration der Luftmischung aufweist, die unterhalb eines vordefinierten Schwellwerts ist,

**dadurch gekennzeichnet, dass** die Vorrichtung ferner aufweist:

einen Beschleunigungsmesser, der geeignet ist, eine Bewegung des Benutzers zu erfassen, wobei der Luftqualitätssensor (9) geeignet ist, kalibriert zu werden, wenn der Beschleunigungsmesser keine Bewegung des Benutzers erfasst, wobei die Vorrichtung (1) eine tragbare Vorrichtung, wie etwa eine Uhr, ist.

2. Vorrichtung nach Anspruch 1, wobei der Hohlraum (5) eine Öffnung (52) aufweist, so dass Luft ausschließlich durch die Öffnung (52) in den Hohlraum (5) eintreten kann, wobei der Hohlraum (5) an anderer Stelle von der Vorrichtung (1) isoliert ist.

3. Vorrichtung (1) nach einem der Ansprüche 1 bis 2, wobei der Luftqualitätssensor (9) geeignet ist, einen elektrischen Strom zu erzeugen, indem er die in dem Innenvolumen des Hohlraums (5) vorhandene Luftverbindung verbraucht.

4. Vorrichtung (1) nach einem der Ansprüche 1 oder 3, wobei die Luftverbindung $NO_2$ ist.

5. Vorrichtung (1) nach einem der Ansprüche 1 bis 4, wobei das Innenvolumen des Hohlraums (3) kleiner als 0,3 $cm^3$, kleiner als 0,2 $cm^3$, kleiner als 0,1 $cm^3$ oder sogar kleiner als 0,05 $cm^3$ ist.

6. Vorrichtung (1) nach einem der Ansprüche 1 bis 5, wobei der Hohlraum (5) ein erstes Gitter (101) und ein zweites Gitter (102) aufweist, wobei das zweite Gitter (102) geeignet ist, sich relativ zu dem ersten Gitter (101) zu bewegen, so dass der Hohlraum (5) in dem offenen Zustand oder in dem geschlossenen Zustand sein kann.

7. Vorrichtung (1) nach Anspruch 6, wobei jedes der ersten und zweiten Gitter (101, 102) eine Vielzahl von Durchgangsöffnungen (103, 104) aufweist, wobei wenigstens einige der Durchgangsöffnungen (103, 104) der ersten und zweiten Gitter (101, 102) einander zugewandt sind, wenn der Hohlraum (5) in dem offenen Zustand ist, und wobei die Durchgangsöffnungen (103, 104) der ersten und zweiten Gitter (101, 102) gegeneinander versetzt sind, wenn der Hohlraum (5) in dem geschlossenen Zustand ist.

8. Vorrichtung (1) nach einem der Ansprüche 1 bis 5, wobei der Hohlraum (5) eine Tür (105) aufweist, wobei die Tür (105) geeignet ist, sich relativ zu der Öffnung (52) zu bewegen, so dass der Hohlraum (5) in dem offenen Zustand oder in dem geschlossenen Zustand sein kann.

**9.** Vorrichtung (1) nach einem der Ansprüche 1 bis 8, wobei der Hohlraum (5) einen unteren Abschnitt (53) und einen oberen Abschnitt (54) aufweist, wobei der untere Abschnitt (53) ein erstes Loch (55) und ein zweites Loch (56) aufweist, wobei der Luftqualitätssensor (9) in dem ersten Loch (55) angeordnet ist, wobei der Hohlraum (5) und der Luftqualitätssensor (9) unter Verwendung wenigstens einer Dichtung (11) in einer luftdichten Weise miteinander versiegelt sind, so dass der Luftqualitätssensor (9) in direktem Kontakt mit der in dem Innenvolumen des Hohlraums (5) vorhandenen Luft ist.

**10.** Verfahren zur Kalibrierung eines Luftqualitätssensors (9) einer Vorrichtung (1) nach einem der Ansprüche 1 bis 9, der wenigstens die folgenden Schritte aufweist:

    - Umschalten des Hohlraums (5) von dem offenen Zustand auf den geschlossenen Zustand;
    - Warten darauf, dass das Innenvolumen die Luftverbindung nicht mehr aufweist oder eine Konzentration der Luftverbindung aufweist, die unter einem vordefinierten Schwellwert ist; und
    - Kalibrieren des Luftqualitätssensors (9).

**Revendications**

**1.** Dispositif (1) comprenant une cavité (5) définissant un volume interne, un capteur de qualité d'air (9) étant adapté pour mesurer une concentration d'au moins un composé de l'air présent dans le volume interne,

    dans lequel la cavité (5) peut être alternativement dans :

        - un état ouvert dans lequel l'air peut entrer dans la cavité (5) depuis l'extérieur de la cavité (5), et
        - un état fermé dans lequel de l'air ne peut pas circuler entre l'extérieur et l'intérieur de la cavité (5),

    le capteur de qualité d'air (9) étant adapté pour être étalonné quand la cavité (5) est dans l'état fermé et dès que le volume interne ne comprend plus le composé de l'air ou comprend une concentration du composé de l'air qui est inférieure à un seuil prédéfini, **caractérisé en ce que** le dispositif comprend en outre un accéléromètre adapté pour détecter un mouvement de l'utilisateur, le capteur de qualité d'air (9) étant adapté pour être étalonné quand l'accéléromètre ne détecte aucun mouvement de l'utilisateur, dans lequel le dispositif (1) est un dispositif pouvant être porté, tel qu'une montre.

**2.** Dispositif selon la revendication 1, dans lequel la cavité (5) comprend une ouverture (52) de sorte que l'air peut entrer exclusivement dans la cavité (5) par l'ouverture (52), la cavité (5) étant par ailleurs isolée du dispositif (1).

**3.** Dispositif (1) selon l'une quelconque des revendications 1 à 2, dans lequel le capteur de qualité d'air (9) est adapté pour produire un courant électrique en consommant le composé de l'air présent dans le volume interne de la cavité (5).

**4.** Dispositif (1) selon l'une quelconque des revendications 1 ou 3, dans lequel le composé de l'air est $NO_2$.

**5.** Dispositif (1) selon l'une quelconque des revendications 1 à 4, dans lequel le volume interne de la cavité (5) est inférieur à 0,3 cm$^3$, inférieur à 0,2 cm$^3$, inférieur à 0,1 cm$^3$, ou même inférieur à 0,05 cm$^3$.

**6.** Dispositif (1) selon l'une quelconque des revendications 1 à 5, dans lequel la cavité (5) comprend une première grille (101) et une seconde grille (102), la seconde grille (102) étant adaptée pour se déplacer par rapport à la première grille (101) de sorte que la cavité (5) peut être dans l'état ouvert ou dans l'état fermé.

**7.** Dispositif (1) selon la revendication 6, dans lequel chacune des première et seconde grilles (101, 102) comprend une pluralité d'ouvertures traversantes (103, 104), dans lequel au moins certaines des ouvertures traversantes (103, 104) des première et seconde grilles (101, 102) se font face quand la cavité (5) est dans l'état ouvert et dans lequel les ouvertures traversantes (103, 104) des première et seconde grilles (101, 102) sont décalées l'une par rapport à l'autre quand la cavité (5) est dans l'état fermé.

**8.** Dispositif (1) selon l'une quelconque des revendications 1 à 5, dans lequel la cavité (5) comprend une porte (105), la porte (105) étant adaptée pour se déplacer par rapport à l'ouverture (52) de sorte que la cavité (5) peut être dans l'état ouvert ou dans l'état fermé.

**9.** Dispositif (1) selon l'une quelconque des revendications 1 à 8, dans lequel la cavité (5) comprend une partie inférieure (53) et une partie supérieure (54), dans lequel la partie inférieure (53) comprend un premier trou (55) et un second trou (56), le capteur de qualité d'air (9) étant situé dans le premier trou (55), la cavité (5) et le capteur de qualité d'air (9) étant scellé ensemble de manière étanche à l'air en utilisant au moins un joint d'étanchéité (11), de sorte que le capteur de qualité d'air (9) est en contact direct avec l'air présent dans le volume interne de la cavité (5).

10. Procédé d'étalonnage d'un capteur de qualité d'air (9) d'un dispositif (1) selon l'une quelconque des revendications 1 à 9, comprenant au moins les étapes suivantes :

- le passage de la cavité (5) de l'état ouvert à l'état fermé ;
- le fait d'attendre que le volume interne ne comprenne plus le composé de l'air ou comprenne une concentration du composé de l'air qui est inférieure à un seuil prédéfini ; et
- l'étalonnage du capteur de qualité d'air (9).

FIG. 1

FIG. 2

FIG. 3

FIG. 4

FIG. 5A

FIG. 5B

FIG. 6

FIG. 7

FIG. 8

**FIG. 9**

**FIG. 10**

FIG. 11

FIG. 12

FIG. 13

**EP 3 650 851 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017351221 A1
- CN 204086834 U
- WO 2017207594 A1
- US 6774613 B1
- US 2011197649 A1
- US 4590789 A